# NEUE EUROPÄISCHE PATENTSCHRIFT

(11) **EP 1 904 026 B2**
(45) Veröffentlichungstag und Bekanntmachung des Hinweises auf die Entscheidung über den Einspruch: **12.03.2014**
(45) Hinweis auf die Patenterteilung: 15.09.2010
(21) Anmeldenummer: 06754488.2
(22) Anmeldetag: 22.06.2006
(51) Int. Cl.: A61K 8/46, A61K 8/44, A61K 8/39, A61K 8/81, A61Q 19/10, A61Q 5/02

(54) **BESONDERS MILDE DUSCHFORMULIERUNG**
ESPECIALLY MILD SHOWER FORMULATION
FORMULATION DE DOUCHE PARTICULIEREMENT DOUCE

(30) Priorität: 12.07.2005 DE 102005033663
(43) Veröffentlichungstag der Anmeldung: 02.04.2008
(73) Patentinhaber: Beiersdorf AG, 20253 Hamburg (DE)
(72) Erfinder: RUPPERT, Stephan, 20259 Hamburg (DE); FRESE, Christian, 22765 Hamburg (DE); KAUFFELDT, Martin, D - 22303 Hamburg (DE)
(74) Vertreter: Hartmann, Jost
(86) Internationale Anmeldenummer: PCT/EP2006/005988
(87) Internationale Veröffentlichungsnummer: WO 2007/006402

(56) Entgegenhaltungen:
- EP-A- 1 470 813
- EP-A- 1 704 898
- WO-A-03/078558
- WO-A-2005/027860
- WO-A-2005/030163
- WO-A1-2005//030163
- DE-A1-102004 027 325
- DE-A1-102004 027 329

## Beschreibung

Die Erfindung betrifft besonders milde Duschformulierungen mit besonderen Schaum- und Schäumeigenschaften.

Bei der Körperreinigung erwartet der Verbraucher ein Duschprodukt, welches einen reichhaltigen Schaum erzeugt, gleichzeitig aber sehr mild ist. Die bekannten Duschprodukte stellen eine Mischung aus Tensiden mit einem ausbalancierten Eigenschaftsprofil dar. Eine Verbesserung der Schaumeigenschaften ist durch die Erhöhung der Tensidmenge möglich, dieses führt aber gleichzeitig zu einer Verringerung der Milde. Eine Erhöhung der Milde des Duschproduktes durch den Einsatz von milden Tensiden führt andererseits zu einer vom Verbraucher nicht akzeptierten Verschlechterung der Schaumeigenschaften.

Die Milde eines Tensidsystems lässt sich durch die Bestimmung der Quotienten aus Hämolysewert und Denaturierungsindex charakterisieren.
Der Quotient UD repräsentiert das Verhältnis von Hämolysewert (L) zu Denaturierungsindex (D) und wird durch den Standard RBC-Test bestimmt. Der Standard RBC-Test dient zur Abschätzung von in-vivo Augenschleimhautreizpotentialen von Tensiden und tensidhaltigen Produkten. Das Verfahren beruht auf der Tatsache, dass Tenside stark mit Zellmembranen und Proteinen interagieren. Beide Effekte werden photometrisch durch Analyse des natürlichen Blutfarbstoffs Oxyhämoglobin (HbO₂) bestimmt. Im Gegensatz zu anderen zellbasierten Systemen ist der RBC-Test in der Lage zwischen Schädigungen der Zellmembran (Hämolyse) und Proteindenaturierung (Denaturierungsindex) zu differenzieren.

Frische Proben von Kälberblut werden direkt vom Schlachthaus bezogen. Die roten Blutzellen werden zur Abtrennung der weißen Blutzellen und von allen Plasmaresten mehrfach gewaschen und zentrifugiert.

### Hämolyse:

Ein definiertes Aliquot isolierter Kalbserythrozyten wird mit einer Reihe steigender Konzentration der zu untersuchenden, waschaktive Substanzen enthaltenden Muster (Stammlösung für Rohstoffe 0,1 Gew.-% Aktivgehalt der waschaktiven Substanz in PBS) für 10 Minuten unter Schütteln bei RT inkubiert. PBS ist ein üblicher Standard-PhosphatPuffer (pH 7,4) mit folgender Zusammensetzung:

| | |
|---|---|
| Na₂HPO₄ * 2 H2O | 3,95 g |
| KH₂PO₄ | 0,76 g |
| NaCl | 7,20 g |
| Glucose * 1 H₂O | 1,80 g |
| Aqua dest. oder "highly purified water" ad 1000 ml | |

Die Inkubationsperiode wird durch schnelle Hochgeschwindigkeitszentrifugation beendet. Nach Zentrifugation werden die gewonnenen Überstände photometrisch auf ihren Gehalt an freigesetztem Hämoglobin (HbO₂) bei 530nm analysiert. Daraus wird der relative Hämolysegrad bezogen auf 100%ige Hämolyse berechnet und der L-Wert [µl/ml] (i. A. auch als H50-Wert bezeichnet) als Kenngröße aus der Konzentrations-Response-Kurve bestimmt. Dieser gibt die Konzentration des Prüfmusters an, bei der 50% des Hämoglobins freigesetzt werden.

### HbO₂-Denaturierungsindex:

Ein definiertes Aliquot isolierter Kalbserythrozyten wird mit einer fixen Konzentration des Prüfmusters (bei Tensiden: 0,1% Aktivgehalt in PBS) für 10 Minuten unter Schütteln bei Raumtemperatur inkubiert und dann schnell zentrifugiert. Die Änderung der spektralen Absorption bei 575 nm und 540 nm wird im Vergleich zum nativen HbO₂ gemessen. Aus dem Verhältnis der Absorptionswerte zueinander wird der Denaturierungsindex D [%] berechnet. Als 100%-Standard dient Na-Laurylsulfat (0,1% Aktivgehalt).

### UD Quotient:

Der Quotient stellt das Verhältnis der Kenngrößen von Hämolyse (L) und Denaturierungsindex (D) dar und wird zur Klassifizierung und Charakterisierung der untersuchten Prüfmuster verwendet.

Das Verfahren ist auch in der INVITTOX-Datenbank zur Niederlegung von Tierversuchsalternativen unter Protokoll Nr. 37 niedergelegt und wird ferner in den folgenden Literaturstellen beschrieben:
Kondo, T. (1976) Mechanisms of haemolysis by surface active agents. Adv. Colloid & Interface Sci., 6, 139-172 Kondo, T. & Tomizawa, M. (1968) Haemolysis by nonionic surface-active agents. J. Pharm. Sci., 57, 1246-1248.
Gloxhuber, Ch. (1974) Toxicological properties of surfactants. Arch. Toxicol., 32, 245-270.
Pape, W.J.W., Pfannenbecker, U. & Hoppe, U. (1987) Validation of the red blood cell test as an in vitro assay for the rapid screening of irritation potential of surfactants. Molecular Toxicology, 1, 525-536.
Pape, W. & Hoppe, U. (1988) Second World Surfactants Congress, Paris. Evaluation of acute irritation potentials of tensides using the in vitro alternative red blood cell test system. Proceedings, IV, 414-428.
Pape, W.J.W. (1990) In vitro methods for the assessment of local effects of cosmetics on skin and mucous membranes. Presented at: In-cosmetics 1990, Birmingham, UK.
Pape, W.J.W. & Hoppe, U. (1991) Standardisation on an in vitro red blood cell test for evaluating the acute cytotoxic potential of tensides. Arzneimittet-Forschung/Drug Research, 40(I), 4, 498-502.
Pape, W.J.W. & Hoppe, U. (1991) In vitro methods for the assessment of primary local effects of topically applied preparations. Skin Pharmacology (in press).

Das Schaumverhalten eines Reinigungsproduktes kann durch nachfolgend beschriebene Schaummessungen bestimmt werden. Weiterhin kann die Schaummenge durch ein Expertenpanel bestimmt werden, wobei sich die Ergebnisse dieser beiden Untersuchungsmethoden decken.

Die Schaummessungen wurden mit einer modifizierten Variante des Ross-Miles Testes (DIN 53 902) durchgeführt. Diese Abwandlung des Testes wurde von
Dr. F.J. Gohlke (Hoechst AG, Frankfurt) in der Zeitschrift Parfümerie und Kosmetik Nr.3/1964, Seiten 59 - 63 beschrieben. Hierbei wird die Versuchsanordnung leicht modifiziert, wodurch sich im Vergleich zur DIN-Vorschrift etwas niedrigere Schaumhöhen ergeben.

Die Modifizierung sieht folgenden Versuchsaufbau vor:
50 ml der zu testenden Tensidlösung, auf 38° temperiert, werden in einen Standzylinder vorgelegt. Aus einer definierten Höhe wird 200 ml Wasser, ebenfalls auf 38° temperiert, mit einem Zusatz von 300 mg/l Calziumsulfat auf die Tensidlösung fallen gelassen. Die hierdurch erzeugte Schaummenge wird sofort, nach 30 Sekunden und nach 1, 3, 5, 10 bzw. 15 Minuten abgelesen und in ein Diagramm eingetragen. Aus den aufgenommenen Daten wird zusätzlich die Schaumstabilität errechnet und ebenfalls grafisch aufgetragen.

Moderne Reinigungsprodukte für die Körperreinigung bestehen im Allgemeinen aus Wasser, Tensiden, Verdicker, Hilfsstoffen und Pflegekomponenten.
Besonders den Tensiden kommt hierbei eine hohe Bedeutung zu, da der Verbraucher eine große Schaummenge und einen feinen Schaum des Produkts und gleichzeitig eine milde Formulierung erwartet. Die Milde eines Duschproduktes kann mit dem Standard RBC-Test bestimmt werden, der zur Abschätzung von in-vivo Augenschleimhautreizpotentialen von Tensiden und tensidhaltigen Produkten eingesetzt wird. Es ist bekannt, dass durch eine deutliche Reduzierung des Tensidgehalts die Milde eines tensidhaltigen Produkts erhöht wird, dies hat jedoch eine Reduzierung der Schaummenge zur Folge. Die Schrift WO 01/97771 A2 offenbart Zubereitungen, enthaltend einen Extrakt der Pflanze Pistia stratiotes. In den Beispielen 23 und 25 finden sich auch tensidhaltige Zubereitungen, die allerdings Schaumbäder darstellen.
Die Schrift WO 01/79414 A1 offenbart Tensidgranulate, dadurch erhältlich, dass man wässrige Pasten aus nichtionischen Tensiden in Gegenwart von organischen polymeren Trägermaterialien granuliert und gleichzeitig trocknet.
Die Schrift WO 01/45650 A2 offenbart die Verwendung von Rückständen aus der Weinherstellung als Wirkstoffzusammensetzungen zur Herstellung von kosmetischen und/oder pharmazeutischen Zubereitungen, insbesondere von Pflegemitteln für Haut und Haare. In den Beispielen 9, 12, 21, 24 und 25 finden sich auch tensidhaltige Zubereitungen, die allerdings sehr hohe Tensidgehalte aufweisen.
Die Schrift US 2003/ 0170265 A1 offenbart die Verwendung von Extrakten des Pilzes Grifola frondosa in kosmetischen und/oder dermatologischen Pflegemitteln für die Haut. In den Beispielen 11 und 13 auf Seite 15 finden sich auch tensidhaltige Zubereitungen, die allerdings sehr hohe Tensidgehalte aufweisen und bei denen es sich um Schaumbäder handelt.
Die Schrift US 6723867 B1 offenbart verzweigte, weitgehend ungesättigte Fettalkoholsulfate, dadurch erhältlich, dass man (a)ungesättigte Fettsäuren mit 16 bis 22 Kohlenstoffatomen in an sich bekannter Weise dimerisiert, (b) die bei der Dimerisierung anfallende Monomerfraktion abtrennt, (c) die in dieser Fraktion enthaltenen verzweigten, weitgehend ungesättigten Fettsäuren in die entsprechenden Fettsäuremethylesterüberführt, (d) die verzweigten, weitgehend ungesättigten Fettsäuremethylester unter Erhalt der Doppelbindungen zu den entsprechenden verzweigten, weitgehend ungesättigten Fettalkoholen hydriert, und diese (e) in an sich bekannter Weise unter Erhalt der Doppelbindung sulfatiert und neutralisiert sowie ihre Verwendung zur Herstellung von Wasch-, Spül-, Reinigungs-und Avivagemitteln und/oder kosmetischen und/oder pharmazeutischen Zubereitungen. In den Beispielen 22, 41, 44 und 45 finden sich auch tensidhaltige Zubereitungen, die allerdings sehr hohe oder sehr niedrige Tensidgehalte aufweisen und bei denen es sich um Schaumbäder oder eine Haarspülung handelt.

Wünschenswert wären Zusammensetzungen von bekannten Tensiden, welche bei sehr guten Schaumeigenschaften eine Milde aufweisen, die die bisherigen Zusammensetzungen deutlich übertrifft.

Es wurde überraschend festgestellt, dass kosmetische Reinigungszubereitungen enthaltend
(a) anionische Schwefelsäureester
(b) amphotheres Tensid
(c) ethoxylierte Glycerin-Fettsäureester mit 3 bis 12 EO-Einheiten, **dadurch gekennzeichnet, dass** die Einsatzkonzentration der amphoteren Tenside mehr als 5,0 Gew.% beträgt und weniger als 8,0 Gew.%, bezogen auf das

Gesamtgewicht der Zubereitung den Nachteilen des Standes der Technik abhelfen. Solche Produkte weisen gegenüber vergleichbaren Marktprodukten eine überragende Milde auf. Gleichzeitig ist aber das Schaumverhalten aussergewöhnlich und überraschend gut. Insbesondere wird ein besonders feiner Schaum erreicht. Sie eignen sich insbesondere als Duschprodukte.
Darüber hinaus war es erfindungsgemäß vorteilhaft, wenn die Einsatzkonzentration von anionischen Sulfattensiden weniger als 9,0 Gew.%, bevorzugt weniger als 8,0 Gew.%, besonders bevorzugt weniger als 6,7 Gew.% und mehr als 4,2 Gew.%, bevorzugt mehr als 4,5 Gew.% beträgt, bezogen auf das Gesamtgewicht der Zubereitung. Hierdurch wird die besondere Milde erreicht.
Ebenfalls erfindungsgemäß ist es, wenn die Gesamtkonzentration an Tensiden weniger als 15 Gew.% sowie mehr als 12,5 Gew.% beträgt, bezogen auf das Gesamtgewicht.
Ebenfalls erfindungsgemäß ist es, wenn die Einsatzkonzentration von niedrig ethoxylierten Glycerin-Fettsäureester mit einem EO-Anteil von 3-12 weniger als 4,0 Gew.%, bevorzugt weniger als 3,0 Gew.%, besonders bevorzugt weniger als 2,5 Gew.%, ganz besonders bevorzugt weniger als 2,2 Gew.% und mehr als 1,8 Gew.% beträgt, bezogen auf das Gesamtgewicht der Zubereitung. Hierdurch wird die Milde weiter gesteigert und das Hautgefühl nach dem Waschen verbessert.
Weiter ist es erfindungsgemäß von großem Vorteil, wenn als anionische Schwefelsäureester Natrium-, Ammonium- oder Magnesiumsalze von Alkylethersulfaten, bevorzugt Natriumsalze von Alkylsulfaten, C₁₂₋₁₃ Parethsulfaten oder Myrethsulfaten eingesetzt werden. Darüber hinaus ist es erfindungsgemäß von großem Vorteil, wenn als amphoteres Tensid Cocamidopropyl Betaine, Sodium Cocoamphoacetate und/oder Disodium Cocoamphoacetate eingesetzt wird. Ebenfalls erfindungsgemäß von großem Vorteil ist es, wenn das der oder die niedrig ethoxylierten Glycerin-Fettsäureester (EO 3-12) aus der Gruppe PEG-10 Olivenfettsäureglyceride, PEG-11 Avocadofettsäureglyceride, PEG-11 Kakaobutterfettsäureglyceride, PEG-9 Kokosfettsäureglyceride, Glycereth-5 Cocoate, PEG-7 Glyceryl Cocoate, PEG-6 Caprylic/Capric Glyceride, besonders bevorzugt aus der Gruppe PEG-10 Olivenfettsäureglyceride (INCI: PEG-10 OLIVE GLYCERIDES), PEG-9 Kokosfettsäureglyceride (INCI: PEG-9 COCOGLYCERIDES), PEG-7 Glyceryl Cocoate gewählt werden. Die erfindungsgemäße Zubereitung weist einen Gehalt an Natrium-Cocoylglutamat und/oder Natrium-Acylglutamat von 0 bis 0,01 Gew.% auf, besonders bevorzugt wenn Natrium-Cocoylglutamat und Natrium-Acylglutamat in der Zubereitung fehlen.

Weiterhin ist es stark bevorzugt, wenn in einer erfindungsgemäßen Zubereitung zusätzlich ein Acrylatverdicker verwendet wird, der aus einem Copolymer mit a) einem Acrylatmonomer ausgewählt aus Acrylsäure, Methacrylsäure, Itaconsäure, Fumarsäure, Crotonsäure, Aconitsäure oder Maleinsäure, b) einem alpha-, beta-ethylenisch ungesättigten Monomer der allgemeinen Formel CH₂=CXY mit X=H, CH₃, -C1-C30-Alkyl, -CH₂-C(=O)O(CH₂-CH₂-O)ₓ-R³, -CH₂-C(=O)NH(CH₂-CH₂-O)ₓ-R³, -CH₂-CH₂=(CH₂-CH₂-O)ₓ-R³ mit x= 1-100 und R³=C1-C30 Alkyl oder Cl und Y= -COOR, -C₆H₄R, -CN, -CONH₂, ₋Cl, -NC₄H₆O, -NH(CH₂)₃COOH, -NHCOCH₃, -CONHC(CH₃)₃, -CON(CH₃)₂, -CH=CH₂, C1-C18-Alkyl, Hydroxy-C1-C18-Alkyl, -C(=O)O(CH₂-CH₂-O)ₓ-R3, -C(=O)NH(CH₂-CH₂-O)ₓ-R³, -CH2=(CH₂-CH₂-O)ₓ-R³ mit x= 1-100 und R³=C1-C30-Alkyl oder der Formel CH₂=CH(OCOR²) mit R²=C1-C18 Alkyl oder der Formel CH₂=CH₂ oder CH₂=CHCH₃ und c) einer mehrfach ungesättigten Komponente, die zur teilweisen Quervernetzung geeignet ist, besteht. Ganz besonders bevorzugt ist es dabe, wenn die Gesamtmenge an einem oder mehreren Acrylatverdickern aus dem Bereich von 0,1 - 8,0 Gew.-%, bevorzugt 0,3 - 6 Gew.-%, insbesondere bevorzugt 0,5 - 4 Gew.-% gewählt wird, bezogen auf das Gesamtgewicht der Zubereitungen.
Ein ganz besonderz bovorzugter Acrylatverdicher ist Aqua-SF1 (INCI: Acrylates Copolymer) erhältlich bei der Gesellschaft Noveon.

Die Duschformulierungen enthalten ferner vorteilhaft Wasser und gegebenenfalls die in der Kosmetik üblichen Zusatzstoffe, z. B. Konservierungsmittel, Konservierungshelfer, Bakterizide, antimikrobielle Stoffe, Parfüme, Farbstoffe, Pigmente, die eine färbende Wirkung haben, Komplexierungs- und Sequestrierungsagentien, Lichtschutzfilter, Perlglanzagentien, Trübungsmittel, Pflanzenextrakte, Vitamine, Wirkstoffe, Verdickungsmittel, Gelbildner, anfeuchtende und/oder feuchthaltende Substanzen, Füllstoffe, die das Hautgefühl verbessern, Fette, Öle, Wachse, oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

### Beispiele

Die Gehalte aller Rohstoffe sind in Aktivgehalten angegeben.

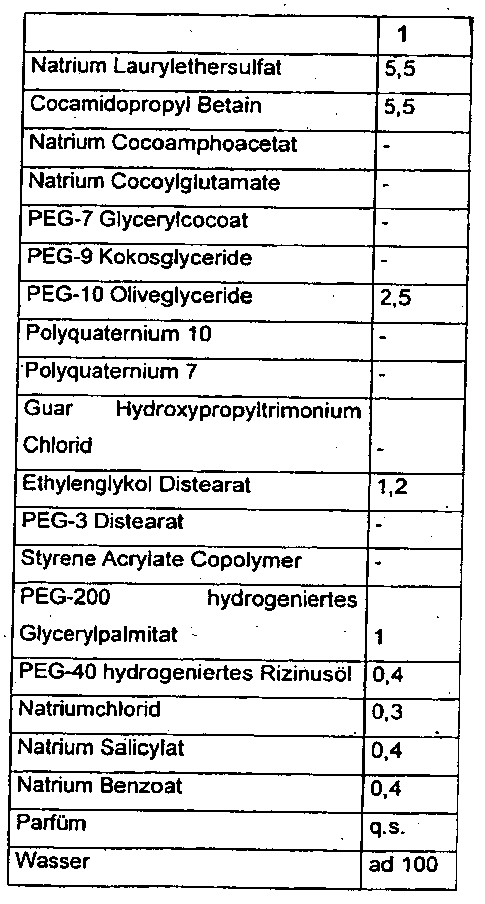

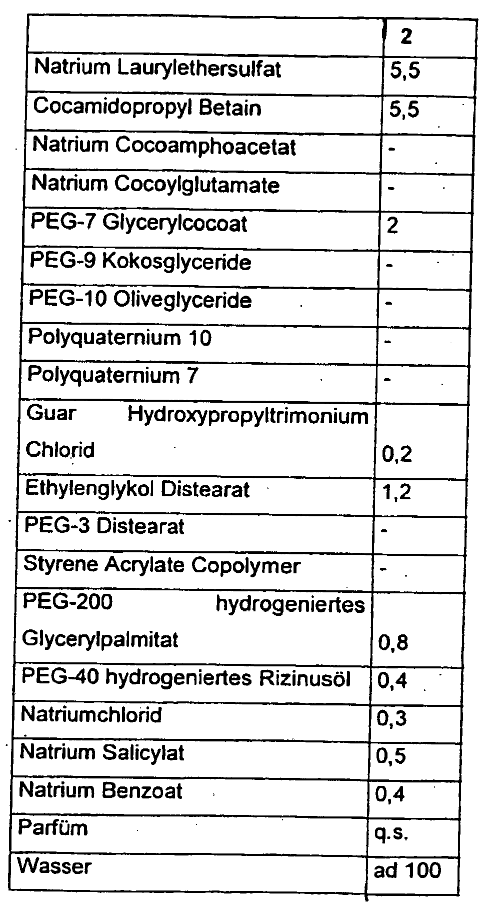

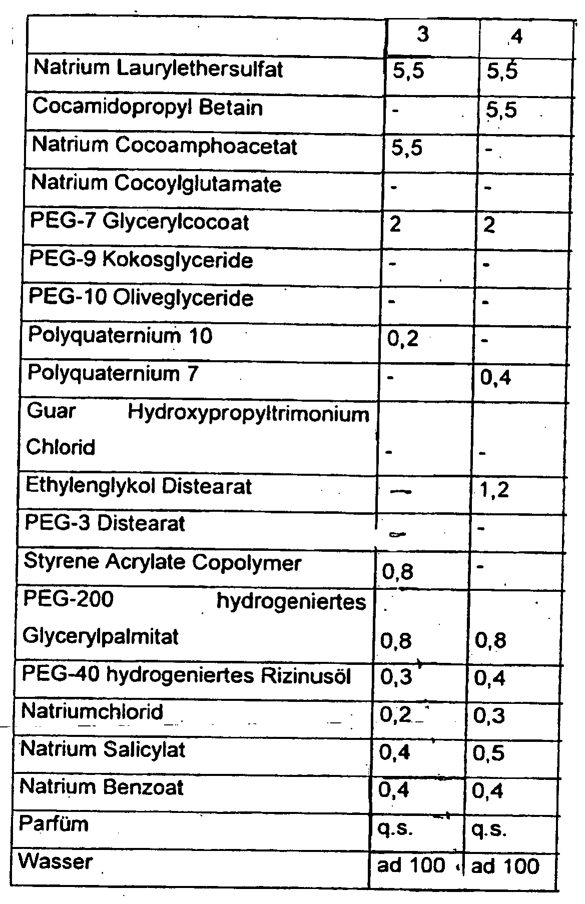

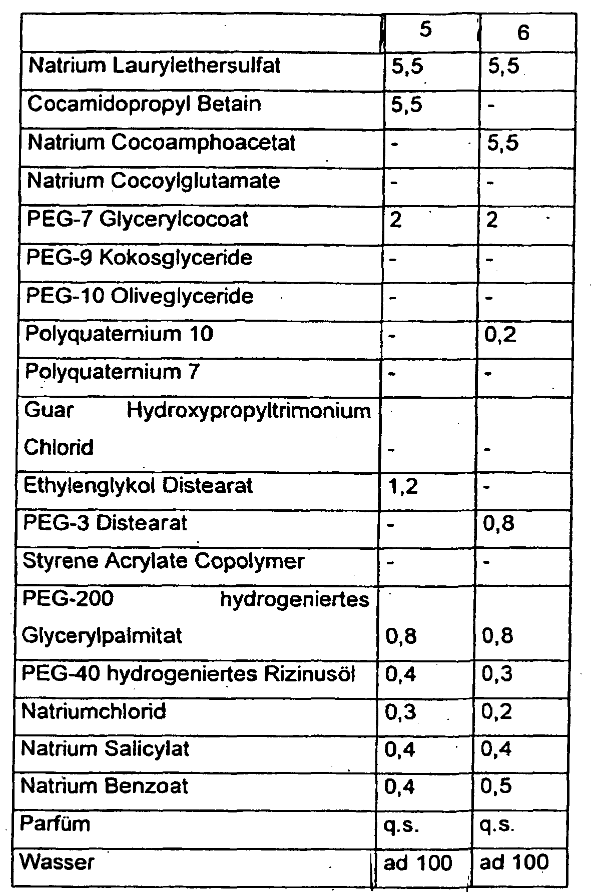

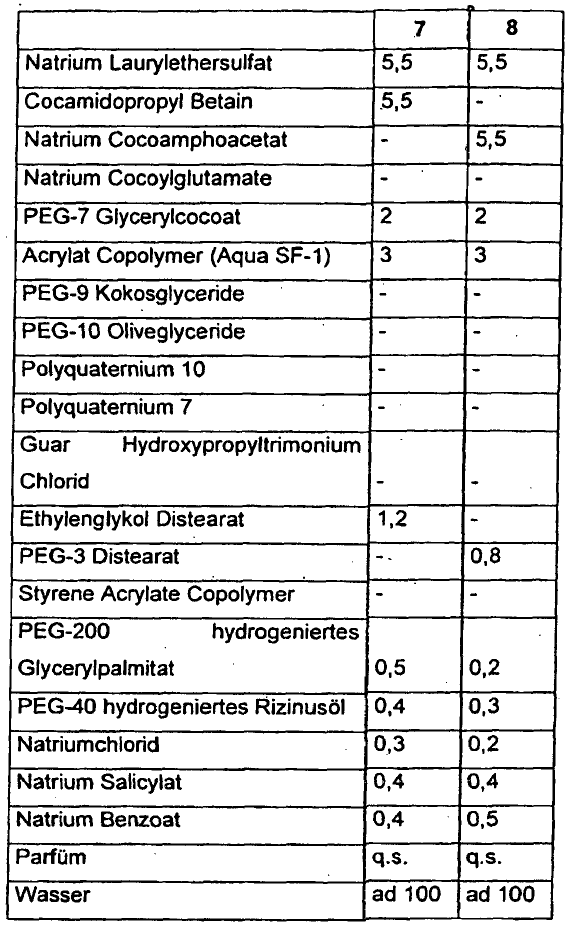

## Patentansprüche

1. Kosmetische Reinigungszubereitungen enthaltend
(a) anionische Schwefelsäureester
(b) amphotheres Tensid
(c) ethoxylierte Glycerin-Fettsäureester mit 3 bis 12 EO-Einheiten,
**dadurch gekennzeichnet, dass** der Gehalt an ethoxylierten Glycerin-Fettsäureester mehr als 1,8 % beträgt und der Gehalt an Tensiden insgesamt weniger als 15 Gew.% sowie mehr als 12,5 Gew.% beträgt, bezogen auf das Gesamtgewicht der Zubereitung, wobei als Tenside auch ethoxylierte Glycerin-Fettsäureester mit 3 bis 12 EO-Einheiten zu verstehen sind und die Einsatzkonzentration von amphoteren Tensiden mehr als 5,0 Gew.% und weniger als 8,0 Gew.%, bevorzugt weniger als 7,0 Gew.%, besonders bevorzugt weniger als 6,0 Gew.% beträgt, bezogen auf das Gesamtgewicht der Zubereitung und der Gehalt an Natrium-Cocoylglutamat und/oder Natrium-Acylglutamat 0 bis 0,01 Gew.% beträgt, besonders bevorzugt **dadurch gekennzeichnet**, das Natrium-Cocoylglutamat und Natrium-Acylglutamat in der Zubereitung fehlen.

2. Zubereitung nach Patentanspruch 1 **dadurch gekennzeichnet, dass** die Einsatzkonzentration von anionischen Sulfattensiden weniger als 9 Gew.%, bevorzugt weniger als 8 Gew.%, besonders bevorzugt weniger als 6,7 Gew.% und mehr als 4,2 Gew.%, bevorzugt mehr als 4,5 Gew.% beträgt, bezogen auf das Gesamtgewicht der Zubereitung.

3. Zubereitung nach einem der vorangehenden Patentansprüche **dadurch gekennzeichnet, dass** als anionische Schwefelsäureester Alkylethersulfate eingesetzt werden.

4. Zubereitung nach einem der vorangehenden Patentansprüche **dadurch gekennzeichnet, dass** als amphoteres Tensid Cocamidopropyl Betain, Sodium Cocoamphoacetat und/oder Dinatrium Cocoamphoacetat eingesetzt wird.

5. Zubereitung nach einem der vorangehenden Patentansprüche **dadurch gekennzeichnet, dass** der oder die niedrig ethoxylierten Glycerin-Fettsäureester aus der Gruppe PEG-10 Olivenfettsäureglyceride, PEG-11 Avocadofettsäureglyceride, PEG-11 Kakaobutterfettsäureglyceride, PEG-9 Kokosfettsäureglyceride, Glycereth-5 Cocoate, PEG-7 Glyceryl Cocoate, PEG-6 Caprylic/Capric Glyceride gewählt werden.

6. Zubereitung nach einem der vorangehenden Patentansprüche **dadurch gekennzeichnet, dass** zusätzlich ein Acrylatverdicker verwendet wird, der aus einem Copolymer mit a) einem Acrylatmonomer ausgewählt aus Acrylsäure, Methacrylsäure, Itaconsäure, Fumarsäure, Crotonsäure, Aconitsäure oder Maleinsäure, b) einem alpha-, beta-ethylenisch ungesättigten Monomer der allgemeinen Formel CH₂=CXY mit X=H, CH₃,-C1-C30-Alkyl, -CH₂-C(=O)O(CH₂-CH₂-O)ₓ-R³, -CH₂-C(=O)NH(CH₂-CH₂-O)ₓ-R³, -CH₂-CH₂=(CH₂-CH₂-O)ₓ-R mit x= 1-100 und R³=C1-C30 Alkyl oder Cl und Y= -COOR,-C₆H₄R, -CN, -CONH₂, -Cl, -NC₄H₆O, -NH(CH₂)₃COOH, -NHCOCH₃, -CONHC(CH₃)₃,-CON(CH₃)₂, -CH=CH₂, C1-C18-Alkyl, Hydroxy-C1-C18-Alkyl, -C(=O)O(CH₂-CH₂-O)ₓ-R³_{,} -C(=O)NH(CH₂-CH₂-O)ₓ-R³, -CH2=(CH₂-CH₂-O)ₓR³ mit x= 1-100 und R³=C1-C30-Alkyl oder der Formel CH₂=CH(OCOR²) mit R²=C1-C18 Alkyl oder der Formel CH₂=CH₂ oder CH₂=CHCH₃ und c) einer mehrfach ungesättigten Komponente, die zur teilweisen Quervernetzung geeignet ist, besteht.

7. Zubereitungen nach Anspruch 6 **dadurch gekennzeichnet, dass** die Gesamtmenge an einem oder mehreren Acrylatverdickern aus dem Bereich von 0,1 - 8,0 Gew.-%, bevorzugt 0,3 - 6 Gew.-%, insbesondere bevorzugt 0,5 - 4 Gew.-% gewählt wird, bezogen auf das Gesamtgewicht der Zubereitungen.

## Claims

1. Cosmetic cleaning preparations comprising
(a) anionic sulphuric acid esters
(b) amphoteric surfactant
(c) ethoxylated glycerol fatty acid esters having 3 to 12 EO units, **characterized in that** the content of ethoxylated glycerol fatty acid esters is more than 1.8%, and the content of surfactants overall is less than 15% by weight, and also more than 12.5% by weight, based on the total weight of the preparation, where ethoxylated glycerol fatty acid esters having 3 to 12 EO units are also to be understood as surfactants and the use concentration of amphoteric surfactants is more than 5.0% by weight and less than 8.0% by weight, preferably less than 7.0% by weight, particularly preferably less than 6.0% by weight, based on the total weight of the preparation and the content of sodium cocoyl glutamate and/or sodium acyl glutamate is 0 to 0.01% by weight, particularly preferably **characterized in that** sodium cocoyl glutamate and sodium acyl glutamate are not present in the preparation.

2. Preparation according to Claim 1, **characterized in that** the use concentration of anionic sulphate surfactants is less than 9% by weight, preferably less than 8% by weight, particularly preferably less than 6.7% by weight and more than 4.2% by weight, preferably more than 4.5% by weight, based on the total weight of the preparation.

3. Preparation according to one of the preceding claims, **characterized in that** alkyl ether sulphates are used as anionic sulphuric acid esters.

4. Preparation according to one of the preceding claims, **characterized in that** cocamidopropyl betaine, sodium cocoamphoacetate and/or disodium cocoamphoacetate is used as amphoteric surfactant.

5. Preparation according to one of the preceding claims, **characterized in that** the glycerol fatty acid ester or esters with a low degree of ethoxylation are selected from the group consisting of PEG-10 olive fatty acid glycerides, PEG-11 avocado fatty acid glycerides, PEG-11 cocoa butter fatty acid glycerides, PEG-9 coconut fatty acid glycerides, glycereth-5 cocoate, PEG-7 glyceryl cocoate, PEG-6 caprylic/capric glyceride.

6. Preparation according to one of the preceding claims, **characterized in that** additionally an acrylate thickener is used which consists of a copolymer with a) an acrylate monomer selected from acrylic acid, methacrylic acid, itaconic acid, fumaric acid, crotonic acid, aconitic acid or maleic acid, b) an alpha-, beta-ethylenically unsaturated monomer of the general formula CH₂=CXY where X=H, CH₃, -C1-C30-alkyl, -CH₂-C(=O)O(CH₂-CH₂-O)ₓ-R³, -CH₂-C(=O) NH(CH₂-CH₂-O)ₓ-R³, -CH₂-CH₂=(CH₂-CH₂-O)ₓ-R³ where x=1-100 and R³=C1-C30 alkyl or Cl and Y=-COOR, -C₆H₄R, -CN, -CONH₂, -Cl, -NC₄H₆O, -NH(CH₂)₃COOH, -NHCOCH₃, -CONHC(CH₃)₃, -CON(CH₃)₂, -CH=CH₂, C1-C18-alkyl, hydroxy-C1-C18-alkyl, -C(=O)O(CH₂-CH₂-O)ₓ-R3, -C(=O)NH(CH₂-CH₂-O)ₓ-R³, -CH2=(CH₂-CH₂-O)x-R³ where x= 1-100 and R³=C1-C30-alkyl or of the formula CH₂=CH(OCOR²) where R²=C1-C18 alkyl or of the formula CH₂=CH₂ or CH₂=CHCH₃ and c) a polyunsaturated component which is suitable for the partial crosslinking.

7. Preparations according to Claim 6, **characterized in that** the total amount of one or more acrylate thickeners is selected from the range 0.1 - 8.0% by weight, preferably 0.3 - 6% by weight, particularly preferably 0.5 - 4% by weight, based on the total weight of the preparation.

## Revendications

1. Préparations cosmétiques de nettoyage, contenant
(a) des esters d'acide sulfurique anioniques
(b) un tensioactif amphotère
(c) des esters d'acides gras avec le glycérol éthoxylés, comportant de 3 à 12 unités OE (oxyde d'éthylène),
**caractérisées en ce que** la teneur en esters d'acides gras avec le glycérol éthoxylés est de plus de 1,8 % et la teneur en tensioactifs est au total inférieure à 15 % en poids, ainsi que supérieure à 12,5 % en poids, par rapport au poids total de la préparation, les esters d'acides gras avec le glycérol éthoxylés comportant de 3 à 12 unités OE, devant également être entendus comme tensioactifs et la concentration utilisée des tensioactifs amphotères étant supérieure à 5,0 % en poids et inférieure à 8,0 % en poids, de préférence inférieure à 7,0 % en poids, de façon particulièrement préférée, inférieure à 6,0 % en poids, par rapport au poids total de la préparation et la teneur en cocoylglutamate de sodium et/ou acylglutamate de sodium allant de 0 à 0,01 % en poids, de façon particulièrement préférée **caractérisée en ce que** le cocoylglutamate de sodium et un acylglutamate de sodium sont absents dans la préparation.

2. Préparation selon la revendication 1, **caractérisée en ce que** la concentration utilisée des tensioactifs de type sulfate anionique est inférieure à 9 % en poids, de préférence inférieure à 8 % en poids, de façon particulièrement préférée inférieure à 6,7 % en poids et supérieure à 4,2 % en poids, de préférence supérieure à 4,5 % en poids, par rapport au poids total de la préparation.

3. Préparation selon l'une quelconque des revendications précédentes, **caractérisée en ce qu'**on utilise comme esters d'acide sulfurique anioniques des alkyléthersulfates.

4. Préparation selon l'une quelconque des revendications précédentes, **caractérisée en ce qu'**on utilise comme tensioactif amphotère la cocosamidopropylbétaïne, le cocoamphoacétate de sodium et/ou le cocoamphoacétate disodique.

5. Préparation selon l'une quelconque des revendications précédentes, **caractérisée en ce que** l'ester ou les esters d'acide gras avec le glycérol faiblement éthoxylé(s) est/sont choisi(s) dans le groupe constitué par les PEG-10 glycérides d'acides gras d'olive, les PEG-11 glycérides d'acides gras d'avocat, les PEG-11 glycérides d'acides gras de beurre de cacao, les PEG-9 glycérides d'acides gras de coco, les glycéréth-5 cocoates, les PEG-7 cocoates de glycéryle, les PEG-6 glycérides caprylique/caprique.

6. Préparation selon l'une quelconque des revendications précédentes, **caractérisée en ce qu'**on utilise en outre un épaississant acrylate, qui consiste en un copolymère comportant a) un monomère acrylate choisi parmi l'acide acrylique, l'acide méthacrylique, l'acide itaconique, l'acide fumarique, l'acide crotonique, l'acide aconitique ou l'acide maléique, b) un monomère à insaturation alpha, bêta-éthylénique de formule générale CH₂=CXY où X = H, CH₃, un groupe alkyle en C₁-C₃₀, -CH₂-C(=O)O(CH₂-CH₂-O)ₓ-R³, -CH₂-C(=O)NH(CH₂-CH₂-O)ₓ-R³, -CH₂-CH₂=(CH₂-CH₂-O)ₓ-R³, avec x = 1-100 et R³ = alkyle en C₁-C₃₀ ou Cl et Y = -COOR, -C₆H₄R, -CN, -CONH₂, -Cl, -NC₄H₆O, -NH(CH₂)₃COOH, -NHCOCH₃, -CONHC(CH₃)₃, -CON(CH₃)₂, -CH=CH₂, alkyle en C₁-C₁₈, hydroxy-alkyle(C₁-C₁₈), -C(=O)O(CH₂-CH₂-O)ₓ-R³, -C(=O)NH(CH₂-CH₂-O)ₓ-R³, -CH₂=(CH₂-CH₂-O)ₓ-R³ avec x = 1-100 et R³ = alkyle en C₁-C₃₀, ou de formule CH₂=CH (OCOR²) où R² = alkyle en C₁-C₁₈ ou de formule CH₂=CH₂ ou CH₂=CHCH₃ et c) un composant polyinsaturé qui est approprié à la réticulation transversale partielle.

7. Préparations selon la revendication 6, **caractérisées en ce que** la quantité totale d'un ou plusieurs épaississants acrylate est choisie dans la plage allant de 0,1 à 8,0 % en poids, de préférence de 0,3 à 6 % en poids, de façon particulièrement préférée de 0,5 à 4 % en poids, par rapport au poids total des préparations.
